# EUROPÄISCHE PATENTANMELDUNG

(11) **EP 2 839 877 A1**
(43) Veröffentlichungstag der Anmeldung: **25.02.2015**
(21) Anmeldenummer: 13181187.9
(22) Anmeldetag: 21.08.2013
(51) Int. Cl.: B01J 13/14, A01N 25/28, A61K 9/50

(54) **Verfahren zur Herstellung von Konzentraten von vorzugsweise wasserlöslichen Wirkstoffen**

(71) Anmelder: Kwizda Agro GmbH, 1010 Wien (AT)
(72) Erfinder: Rauch, Andreas, 1220 Wien (AT); Mühlanger, Andreas, 3713 Reinprechtspölla (AT)
(74) Vertreter: Sonn & Partner Patentanwälte

(57) **Zusammenfassung**

Beschrieben wird ein Verfahren zur Herstellung von Konzentraten von vorzugsweise wasserlöslichen Wirkstoffen, wobei in einem wasserfreien Verfahren unter Verwendung fester Wirkstoffe als Ausgangsmaterial die Kristalle eines Wirkstoffs in einem ersten organischen Lösungsmittel unter Zugabe eines Dispergierhilfsmittels gleichmäßig verteilt werden, gegebenenfalls die Viskosität der so erhaltenen Lösung mit einem geeigneten Hilfsmittel eingestellt wird, zu der derart erhaltenen Lösung ein Polymerbildner, gegebenenfalls in einem zweiten organischen Lösungsmittel, zugegeben wird, wobei die Viskosität entweder der zuzugeben oder der erhaltenen Lösung gegebenenfalls durch Zugabe eines geeigneten Hilfsmittels eingestellt wird, und der erhaltenen Lösung ein Vernetzungsmittel mit mindestens zwei funktionellen Gruppen in einem dritten organischen Lösungsmittel zurückgegeben wird, wobei wiederum die Viskosität entweder der zuzugeben oder der erhaltenen Lösung gegebenenfalls durch Zugabe eines geeigneten Hilfsmittels eingestellt wird.

## Beschreibung

Die vorliegende Erfindung betrifft ein Verfahren zur Einkapselung eines festen, vorzugsweise wasserlöslichen Wirkstoffs oder Wirkstoffgemischs durch Vorsehen einer Beschichtung, wobei durch die Beschichtung die Wasserlöslichkeit der beschichteten Partikel in definierter Weise zumindest reduziert, vor allem aber zeitlich so lange verzögert wird, bis der gewünschte Effekt des Wirkstoffs oder Wirkstoffgemischs erreicht wurde.

Zahlreiche Wirkstoffe liegen in kristalliner Form vor und weisen eine relativ geringe Wasserlöslichkeit im Bereich einiger Gramm pro Liter, typischerweise 0,1 bis 50 g/l auf. Diese Eigenschaft erschwert oder verhindert sowohl die Formulierung in Form von stabilen Suspensionen, indem es zur Rekristallisation bei Temperaturänderungen kommt, als auch eine Anwendung unter feuchten Bedingungen, weil es nach der Ausbringung durch Niederschläge, Bodenfeuchte, Reinigungsvorgänge etc. zu einer Auswaschung (sogenanntes "Leaching") der Wirkstoffe kommt. Dies führt zu verminderter Wirkung sowie unerwünschtem Eintrag von Wirkstoffen beispielsweise in Gewässer und in der Folge möglicherweise zu Umweltbeeinträchtigungen.

Bisher wurde diesem Problem begegnet, indem Formulierungen durch mechanische Kompaktierung (z.B. Verpressung), Coating in der Wirbelschicht oder in Dragierkesseln mit Wachsen, Harzen und Polymeren, durch Mikroverkapselung in flüssiger Phase oder Matrixverkapselung hergestellt werden. Diese bekannten Verfahren weisen jedoch zahlreiche wesentliche Mängel auf.

So kann durch mechanische Kompaktierung die Löslichkeit der Wirkstoff nicht wesentlich beeinflusst werden, es wird durch Härtung der Oberfläche lediglich die Wasseraufnahme verzögert.

Eine Beschichtung in der Wirbelschicht oder in Dragierkesseln durch Aufsprühen von gelösten oder geschmolzenen Harzen, Wachsen oder Polymeren führt in der Regel zu einer relativ inhomogenen Verteilung der Coatingmasse über die in Pulverform vorgelegten Wirkstoffe. Aufgrund des gegenseitigen Kontaktes der Partikel in der durch Lufteinblasung oder mechanisch bewegten Schicht kommt es in weiterer Folge zu einem Gleichgewichtszustand zwischen Abrieb und Auftrag, sodass die auf die Wirkstoffe aufbringbare Beschichtungsstärke insgesamt limitiert ist.

Eine Mikroverkapselung im wässrigen Milieu scheitert bei der Verwendung von wasserlöslichen Wirkstoffen, da bei Kontakt der inneren, meist hydrophoben Phase zur äußeren, wässrigen Phase die Wirkstoffe zumindest teilweise aufgelöst werden und sich so der Verkapselung entziehen.

Bei Umkehrphasenverkapselung, wo die innere Phase die wässrige und die äußere Phase die ölige darstellt, ist es notwendig, die Wirkstoffe entweder in einem mit Wasser nicht mischbaren Lösungsmittel zu lösen, wodurch es zur Limitierung der erreichbaren maximalen Konzentration kommt, oder die Wirkstoffe müssen in geschmolzener Form vorliegen, was nicht immer möglich oder erwünscht ist. Darüber hinaus kann es bei Rekristallisation der geschmolzenen Wirkstoffe nach der Verkapselung durch die Formänderung zu einem Platzen der Kapsel kommen, wodurch der Schutz weitgehend verloren geht.

Bei einer Matrixverkapselung, wo die Wirkstoffpartikel in fester Form durch eine äußere kontinuierliche Phase eingeschlossen werden und anschließend durch Brechen, Reiben oder Mahlen mechanisch nachbehandelt werden müssen, werden durch eben diesen Zerkleinerungsvorgang die zuvor geschützten Partikel logischer Weise teilweise wieder freigelegt, wodurch ebenfalls der Schutz weitgehend verloren geht.

Die DE 10 2010 028826 A1 offenbart ein Verfahren zur Herstellung wirkstoffhaltiger Mikrokapseln unter Verkapselung des jeweiligen Wirkstoffs durch Polymerisationsreaktion, wobei die Mikrokapseln auf Epoxidharzen beruhen und der Wirkstoff unter Reaktionsbedingungen entweder als Flüssigkeit oder aber auch als Feststoff vorliegen kann.

Aus der EP 0 841 088 A2 sind Mikrokapseln bekannt, deren Wände aus Umsetzungsprodukten von Aminogruppen enthaltenden Vernetzern mit Isocyanaten bestehen bzw. solche Umsetzungsprodukte enthalten. Die derartig hergestellten Mikrokapseln, welche üblicherweise einen Durchmesser im Bereich von 3-25 µm aufweisen, werden für kohlefreies Durchschreibepapier verwendet. Wie üblich basiert das verwendete Verfahren auf dem chemischen Prinzip der Grenzflächenpolymerisation bzw. Grenzflächenpolyaddition, wobei die zu verkapselnden Stoffe in einem hydrophoben Öl gelöst, mit einem zur Wandbildung befähigten Polyisocyanat versetzt und anschließend mit Wasser zu einer Öl-in-Wasser-Emulsion verarbeitet werden. Diese Emulsion wird dann mit den Vernetzern versetzt, wodurch an der Grenzfläche zwischen Öl und Wasser ein Polymerfilm gebildet wird. Die möglichen Verwendungen von Di- oder Polyaminen, Diolen, Polyolen bzw. politikfunktionellen Aminoalkoholen als Vernetzer werden erwähnt. Ein ähnlicher Stand der Technik ist auch aus der DE 10 2004 059977 A1 bekannt, wobei die hergestellten Mikrokapseln in elektrophoretischen Bildanzeigevorrichtungen verwendbar sind. Auch hier kommt das Prinzip der Grenzflächenpolymerisation bzw. Grenzflächenpolyaddition zum Einsatz, wobei eine Öl-in-Wasser-Emulsion verarbeitet wird, ebenso wie im Verfahren gemäß DE 10 2010 028826 A1.

Es versteht sich, dass diese bekannten Techniken für wasserlösliche Wirkstoffe nicht oder nur sehr beschränkt verwendbar sind.

Nächstkommender Stand der Technik für den Gegenstand der vorliegenden Erfindung ist die US 5 911 923 B1 (Work et al), welche die mögliche Einkapselung eines festen Wirkstoffs offenbart. Das Dokument betrifft ein Verfahren zur Mikroverkapselung eines Wirkstoffs, worin der Wirkstoff in einer organischen kontinuierlichen Phase dispergiert und ein polyfunktionelles Isocyanat und ein Diol oder Polyol in Gegenwart eines optionalen Polymerisationskatalysators zugegeben wird. Geeignete Isocyanate werden beschrieben, darunter auch Phenylendiisocyanate, auch werden geeignete Diole genannt.

Aufgabe der vorliegenden Erfindung ist es nun, Konzentrate von vorzugsweise wasserlöslichen Wirkstoffen vorzusehen, welche Wirkstoffe in fester, kristalliner oder mikrokristalliner Form oder in an amorphe, mikroporöse Partikel gebundener Form derart beschichtet sind, dass jedes einzelne Partikel mit einer definierten, gleichmäßig dicken Schutzschicht überzogen ist, wodurch eine definierte, reproduzierbar dauerhafte Hydrophobierung erreicht wird. Wenn im Folgenden der Begriff "Wirkstoff" verwendet wird, so wird darunter auch ein Gemisch verschiedener Wirkstoffe verstanden. Gleichermaßen wird unter dem Begriff "Kristalle eines Wirkstoffs" ein Wirkstoff oder Wirkstoffgemisch in fester, kristalliner oder mikrokristalliner Form oder in an amorphe, mikroporöse Partikel gebundener Form verstanden.

Die erfindungsgemäße Lösung besteht darin, in einem wasserfreien Verfahren unter Verwendung fester Wirkstoffe als Ausgangsmaterial die Kristalle eines Wirkstoffs in einem ersten organischen Lösungsmittel unter Zugabe eines Dispergierhilfsmittels gleichmäßig zu verteilen, gegebenenfalls die Viskosität der so erhaltenen Lösung mit einem geeigneten Hilfsmittel einzustellen, zu der derart erhaltenen Lösung einen Polymerbildner, gegebenenfalls in einem zweiten organischen Lösungsmittel, zuzugeben, wobei die Viskosität entweder der zuzugebenden oder der erhaltenen Lösung gegebenenfalls durch Zugabe eines geeigneten Hilfsmittels eingestellt wird, und der erhaltenen Lösung ein Vernetzungsmittel mit mindestens zwei funktionellen Gruppen in einem dritten organischen Lösungsmittel zuzugeben, wobei wiederum die Viskosität entweder der zuzugebenden oder der erhaltenen Lösung gegebenenfalls durch Zugabe eines geeigneten Hilfsmittels eingestellt wird. Die durch Zugabe eines Dispergierhilfsmittels erhaltene Suspension wird vorzugsweise durch geeignete Rühr- oder/und Dispergierwerkzeuge homogen gehalten, während der Polymerbildner so langsam zudosiert wird, dass es hierbei zu keiner lokalen Reaktion in Form von Aggregationen (Klumpenbildung) kommt. Falls erforderlich, kann der Polymerbildner auch mit geeigneten Lösungsmitteln verdünnt oder/und mit Hilfe hierfür geeigneter Dispergierhilfsmittel emulgiert werden. Die mit dem Vernetzungsmittel versetzte Suspension wird so lange auf Reaktionstemperatur gehalten und intensiv weiter gerührt, bis der Polykondensationsprozess weitgehend abgeschlossen ist. Dies ist der Fall, sobald die gebildeten beschichteten Kristalle ihre oberflächliche Klebrigkeit verloren haben und demzufolge nach Abschalten der Rührwerkzeuge nicht mehr miteinander agglomerieren und eine frei fließende, klumpenfreie Suspension erhalten bleibt. Diese Suspension, welche die nunmehr fertig beschichteten, diskreten Kristalle des Wirkstoffs enthält, wird auf Raumtemperatur abgekühlt und kann durch Zugabe geeigneter Dispergier- und Stabilisierungsmittel in ein verkaufsfähiges Produkt überführt, oder aber gegebenenfalls weiteren Verfahrensschritten zum Erhalt eines trockenen Pulvers oder Granulates unterzogen werden. Für die direkte Verwendung der Suspension haben sich als besonders geeignete Stabilisierungsmittel pyrogene Kieselsäuren vom Type Aerosil^{®} 200 der Firma Evonik sowie gefällte Kieselsäuren vom Typ Sipernat^{®} 22 der Firma Evonik erwiesen. Falls eine spätere spontane Emulgierung in Wasser erfolgen soll, ist die Zugabe geeigneter Emulgatoren erforderlich. Hierfür haben sich ethoxylierte C₁₁-C₁₅ technische Alkohole, beispielsweise vom Typ Tergitol^{®} 15 S 7 der Firma Dow, Polyoxyethylen-oleylether der Typen Brij^{®} 05 und O10 der Firma Croda, sowie Polyethylenglycolester von Fettsäuren der Type Geronol^{®} VO 2003 der Firma Rhodia als besonders geeignet erwiesen. Für die Überführung der Suspension in eine trockene Formulierung kommen grundsätzlich im Stand der Technik bekannte Absorptions- und Trocknungsprozesse in Frage, insbesondere die Vakuumtrocknung, die Sprühtrocknung, sowie die Wirbelschichttrocknung und Wirbelschichtgranulierung, falls erforderlich unter Hinzufügung geeigneter Schutzkolloide, Bindemittel, Netzmittel und Füllstoffe, jeweils optional mit vorgeschalteter teilweiser Verdampfung und zumindest partieller, besser vollständiger Lösungsmittelrückgewinnung. Das gesamte erfindungsgemäße Verfahren kann atmosphärisch offen, aber auch je nach Dampfdruck und Flammpunkt des gewählten Lösungsmittels teilweise oder vollständig unter Schutzgas bzw. bei Überdruck oder Unterdruck stattfinden, wobei die erfindungsgemäß bevorzugte Ausführungsform die in-situ Beschichtung bei Atmosphärendruck unter Vermeidung einer explosionsgefährlichen Atmosphäre durch Wahl hierfür geeigneter Lösungsmittel darstellt. Eine mögliche, aber nicht zwingend erforderliche zusätzliche Beschleunigung der Polykondensation durch Anwendung physikalischer Methoden wie kontinuierlicher Mischverfahren, Dünnschichtreaktoren, UV Bestrahlung oder Ultrabeschallung ist ebenfalls ausdrücklich Bestandteil der Erfindung.

Geeignete Substrate für die Beschichtung sind grundsätzlich solche Wirkstoffe, die bei Reaktionstemperatur nicht in geschmolzener Form vorliegen und eine mit dem Polymerbildner kompatible Oberfläche aufweisen, das heißt von diesem chemisch nicht angegriffen werden. Auch mehrschichtig aufgebaute Partikel sowie mikroporöse Partikel mit gegebenenfalls darin absorbierten Wirkstoffen kommen als Substrate in Frage. Als Wirkstoffe werden hier allgemein biologisch wirksame, im Speziellen insektizide, herbizide, fungizide, akarizide, algizide, mikrobizide, mikrobistatische, rodentizide, antibiotisch wirksame Wirkstoffe, sowie Repellentien, Attraktoren, Pheromone und allgemein attraktiv oder abwehrend wirkende Duft- oder Geschmacksstoffe sowie Mischungen derartiger Wirkstoffe verstanden. Vorzugsweise wird der Wirkstoff vor der Beschichtung auf eine für die in-situ Polykondensation günstige Partikelgröße von 1-500 µm, vorzugsweise 3-50 µm, gebracht. Dies kann je nach den physikalisch-chemischen Eigenschaften, wie Schmelzpunkt, Siedepunkt, Härte, Mindestzündenergie, Brennbarkeit, spezifischer Leitfähigkeit etc., durch Trocken- oder bevorzugt durch Nasszerkleinerung, etwa in einer Stiftmühle, Luftstrahlmühle oder Kugelmühle, erreicht werden.

Das Dispergierhilfsmittel dient dabei dazu, die Kristalle des verwendeten Wirkstoffs im ersten organischen Lösungsmittel zueinander in Abstand zu halten, um die nachfolgende Einkapselung der Kristalle des Wirkstoffs zu ermöglichen bzw. zum Emulgieren des Polymerbildners im gegebenenfalls verwendeten zweiten organischen Lösungsmittel. Als besonders geeignete Dispergierhilfsmittel haben sich hierbei Polyvinylpyrrolidonderivate, insbesondere der Firma ISP, speziell in Form von Agrimer^{®} AL 22 und Surfadone^{®} LP 100 oder 300, erwiesen. Das Dispergierhilfsmittel muss dabei natürlich mit den verwendeten Lösungsmitteln kompatibel sein.

Die verwendeten Lösungsmittel müssen eine bestimmte, geringe Viskosität aufweisen, der Wirkstoff bzw. gegebenenfalls die Mischung an Wirkstoffen müssen in den Lösungsmitteln unlöslich sein, die Lösungsmittel dürfen keine Hydroxyl- und/oder Aminogruppen aufweisen und der Polymerbildner bzw. das Vernetzungsmittel müssen in ihren jeweiligen Lösungsmitteln löslich sein. Die gestellten Anforderungen können auch durch ein einziges Lösungsmittel erfüllt werden, d.h., dass das erste, zweite und dritte organische Lösungsmittel ident sein kann. Unter den hierfür in Frage kommenden Stoffen haben sich speziell Ester von Pflanzenölen, insbesondere Methylester und Ethylhexylester der mittelkettigen gesättigten, allenfalls einfach oder mehrfach ungesättigten Fettsäuren mit 8 bis 16 C-Atomen, jedoch ohne reaktive Hydroxylgruppen, erwiesen. Derartige geeignete Lösungsmittel kommen z.B. unter dem Handelsnamen Radia^{®} 7118 der Firma Oleon in den Handel. In solchen Fällen, wo diese bevorzugten Lösungsmittel aufgrund ihrer Lösungseigenschaften für den zu beschichteten Wirkstoff nicht in Frage kommen, können auch andere Lösungsmittel, wie Terpenkohlenwasserstoffe, insbesondere Orangenterpen, aber auch aliphatische und aromatische Kohlenwasserstoffe, sowie Ether und Ester natürlicher und technischer aliphatischer und aromatischer Alkohole in verzweigter oder unverzweigter Form sowie Mischungen solcher Lösungsmittel verwendet werden. Hingegen haben sich ein- und mehrwertige Alkohole, Polyole, Amine, Quats und generell stark polare oder ionische Lösungsmittel aufgrund ihrer Reaktionsfähigkeit mit dem Polymerbildner als ungeeignet erwiesen. Vorzugsweise handelt es sich bei dem organischen Lösungsmittel um Surfadone^{®} LP 300, Orangenterpen und/oder Tributylcitrat.

Vorzugsweise wird der Polymerbildner in der für die gewünschte Beschichtungsstärke erforderlichen Menge zugegeben. Diese Menge richtet sich nach der Konzentration der Suspension an Wirkstoff, der Partikelgröße sowie der für die Hydrophobierung notwendigen Schichtdicke. Das Mengenverhältnis zwischen Wirkstoff und Polymerbildner kann, ausgedrückt in Massenprozent bezogen auf die Wirksubstanz von 0,1% bis 500%, bevorzugt jedoch 15% bis 100%, betragen. Bevorzugte Polymerbildner sind niedrigviskose Polymethylen-Polyphenylisocyanate vom Typ "polymeric MDI" mit einem mittlerem NCO Gehalt (25-35, besonders bevorzugt 30 - 32%), wie zum Beispiel die Produkte Voranate M220 von Dow Chemical sowie Suprasec 5025 von Huntsman. Auch Toluoldiisocyanate (TDI) wurden als geeignete Polymerbildner identifiziert, wobei auch hier den niedrigviskosen Typen mit geringer Acidität, wie zum Beispiel dem Produkt Voranate T-80 von Dow Chemical, der Vorzug zu geben ist. Höher viskose Produkte müssen zuvor mit den erwähnten organischen Lösungsmitteln verdünnt werden, wodurch ein jedoch ungünstigeres Mischungsverhältnis zwischen Wirkstoffkonzentration und Konzentration an Polymerbildner entsteht.

Vorzugsweise findet die erfindungsgemäße Umsetzung bei zwischen 10 und 80, vorzugsweise zwischen 40 und 60°C statt, bzw. bei einer Temperatur von mindestens 1 °C, besser mindestens 5 °C, besonders bevorzugt mehr als 10°C unterhalb des Flammpunktes des jeweils verwendeten organischen Lösungsmittels oder/und einer Temperatur, bei welcher das bzw. die verwendete(n) Lösungsmittel einen derart geringen Dampfdruck aufweisen, dass bei Reaktionsbedingungen keine explosionsgefährliche Atmosphäre entsteht.

Gemäß einer bevorzugten Ausführungsform der vorliegenden Erfindung ist das Vernetzungsmittel ein zumindest bifunktioneller Quervernetzer für NCO Gruppen und wird in zumindest stöchiometrischer Menge zugegeben, sodass sämtliche NCO Gruppen des Polymerbildners vernetzt werden, wodurch sich auf der Oberfläche der Wirkstoffpartikel in situ eine Polyurethanschicht bildet. Laut Literatur wären geeignete Reaktionspartner für NCO Gruppen mehrwertige Alkohole, Polyole, Di-, Tri- und Polyamine sowie Mischungen daraus, die im gewählten Lösungsmittel löslich sind und mit diesem chemisch nicht reagieren. Als besonders geeignete Reaktionspartner haben sich jedoch überraschenderweise Ethanolamine, insbesondere Triethanolamin (TEA) erwiesen. Aus der Literatur bekannte Reaktionspartner, wie DETA (Diethylentriamin), EDA (Ethylendiamin), TEDA (Triethylendiamin), HMD (Hexamethylendiamin), können grundsätzlich ebenfalls verwendet werden, führen aber zu weniger günstigen Eigenschaften der Polyurethanschichten, insbesondere zu einer geringeren mechanischen Festigkeit.

Die vorliegende Erfindung wird nun anhand der nachfolgenden Beispiele näher erläutert, auf welche sie jedoch nicht beschränkt ist.

### Beispiel 1 (Grundlage)

In den folgenden Schritten wird eine Beschichtung von Acetamiprid-Kristallen mit einer hydrophobierenden Schicht aus Polyurethan beschrieben.
I. 50 g trocken gemahlenes Acetamiprid mit einer durchschnittlichen Partikelgröße d50 von 5-6 µm wird in eine Lösung aus 142 g Radia^{®} 7118 (Methyllaurat) von Oleon N.V. (NL) und 2,9 g Agrimer^{®} AL 22 (ein alkyliertes Polyvinylpyrrolidon) von ISP International Specialty Products (US) bei Raumtemperatur mit einem hochscherenden Mixer eindispergiert und anschließend auf 50 °C erhitzt.
II. Eine Mischung bestehend aus 9,4 g Voranate^{®} M 220 (Polymethylenpolyphenylisocyanat) von The Dow Chemical Company (US) und 3,5 g Surfadonee^{®} LP 300 (lineares N-Alkyl-2-Pyrrolidon) von ISP International Specialty Products (US) wird dann langsam der Dispersion zugetropft und die Mischung anschließend für 10 Minuten gerührt.
III. Aus 3,7 g Triethanolamin (TEA) und 9,4 g Surfadonee^{®} LP 300 wird eine Lösung hergestellt und diese ganz langsam in einem Zeitraum von etwa 60 Minuten zugetropft.
IV. Für eine ausreichende Reaktionsgeschwindigkeit wird die Temperatur zwischen 50 und 60 °C gehalten. Nach etwa 10 Minuten beginnt die Reaktion zu starten, indem die Lösung anfängt zu verdicken. Um ein übermäßiges Verdicken der Suspension während der Beschichtungsreaktion zu vermeiden, wird die Lösung mit einem Ultra Turrax^{®} Homogenisiersystem von IKA Werke GmbH & Co. KG (DE) intensiv gerührt. Unzureichendes Rühren während der Reaktion führt zur Bildung von Polymerklumpen, was eine inhomogene Beschichtung zur Folge haben kann.
V. Die Dispersion wird dann noch 2 h bei 50°C gerührt und anschließend wieder auf Raumtemperatur abgekühlt.

Um den Grad bzw. die Qualität der Beschichtung zu bestimmen, wird die hergestellte Dispersion in eine flüssige Bautenschutzfolie auf Wasserbasis in einer derartigen Menge eindispergiert, dass der Gehalt an Acetamiprid in der fertigen Folie 2 g/kg beträgt.

4 g dieser flüssigen Folie, entsprechend 8 mg beschichtetem Acetamiprid, werden danach in 100 ml Wasser extrahiert und der Gehalt an Acetamiprid via HPLC-UV bestimmt.

Folgende Ergebnisse wurden ermittelt.

| Extraktionszeit | Acetamiprid in Lösung mg/100ml | Acetamiprid gelöst |
|---|---|---|
| 30 Minuten | 1,04 | 13% |
| 24 Stunden | 2,40 | 30 % |
| 48 Stunden | 2,62 | 33 % |
| 7 Tage | 2,96 | 37 % |

Normalerweise besitzt Acetamiprid laut Literatur eine Löslichkeit bei 20°C von 400 mg/100ml. Aus den in der obigen Tabelle angeführten Ergebnissen wird deutlich, dass die Löslichkeit durch die Beschichtung der Kristalle wesentlich reduziert wurde.

### Beispiel 2 (Steuerung der Hydrophobierung über die Menge an Polymerbildner)

In diesem Beispiel wird die Menge an Polymerbildner im Vergleich zu Beispiel 1 verdoppelt, weiters wurde die Beschichtung in Orangenterpen als Lösungsmittel durchgeführt.
I. 50 g gemahlenes Acetamiprid mit einer durchschnittlichen Teilchengröße von 5-6 µm wird in eine Lösung bestehend aus 135 g Orangenterpen und 2,5 Agrimer^{®} AL 22 bei Raumtemperatur mit Hilfe eines hochscherenden Mixers eindispergiert und anschließend wird die Lösung auf 45°C erhitzt
II. **25,2 g Voranate^{®} M 220 werden mit 25,2 g Citrofol^{®} B1 (Acetyltributyl Citrate) von Jungbunzlauer (AT) gemischt und langsam der Dispersion zugetropft.**
III. 9,2 g Triethanolamin werden unter laufenden hochscherenden Mixer der Dispersion in einem Zeitraum von 60 Minuten langsam zugetropft. Die Dispersion bleibt ständig in Bewegung.
IV. Die Reaktion ist dann beendet, wenn die Lösung bei abgeschaltetem Rührwerk nicht mehr verdickt. Das ist nach weiteren 30 Minuten Rühren der Fall.
V. Nach dem Ende der Reaktion wird die Dispersion für 2 Stunden bei 40°C gerührt.

Die Qualität der Beschichtung wurde mit der gleichen Methode wie im Beispiel 1 beschrieben überprüft

| Extraktionszeit | Acetamiprid in Lösung mg/100ml | Acetamiprid gelöst |
|---|---|---|
| 30 Minuten | 0,40 | 5% |
| 24 Stunden | 0,72 | 9 % |
| 48 Stunden | 0,80 | 10 % |
| 7 Tage | 1,20 | 15 % |

In diesem Fall bewirkt die Verdoppelung des Polymers auch eine Verdoppelung der Hydrophobierung der Wirkstoffkristalle. Ebenfalls kann mit diesem Beispiel gezeigt werden, dass die Beschichtung in einem andern Lösungsmittelmedium funktioniert.

### Beispiel 3 (Abgrenzung des Mengenbereiches)

Hier wird die Menge an Polymerbildner im Vergleich zu Beispiel 1 um 50 % reduziert.

Die Durchführung dieses Experimentes entspricht dem Beispiel 1. Jedoch wird hier die Menge an Voranate^{®} M 220 von 9,2 g auf 4,6 g und die Menge an Triethanolamin von 3,7 g auf 1,85 g reduziert.

Qualitätstests wie für Beispiel 1 und 2 beschrieben ergaben, dass nach 30 Minuten bereits 100 % des Acetamiprids in Lösung waren. Die Beschichtung ist somit unzureichend.

### Beispiel 4 (Abgrenzung des Temperaturbereiches)

Mengen und Reagenzien entsprechen denen von Beispiel 1, jedoch wird hier der gesamte Ansatz gekühlt, um die Temperatur während des Beschichtungsvorganges konstant unter 15°C zu halten. Das Resultat daraus ist, dass die Reaktion erst nach 2 Stunden startet und sehr langsam verläuft. Außerdem verdickt die Lösung trotz intensiven Rührens sehr stark, sodass eine kompakte Paste entsteht.

Der Grund für dieses Verhalten ist die steigende Viskosität der Dispersion bei niedrigen Temperaturen, was zu einem Zusammenklumpen der Teilchen führt. Für das Durchführen der Beschichtung bei niedrigen Temperaturen sind daher Dispersionsmedien mit geringer Viskosität bevorzugt.

Die Qualität der erhaltenen Kapsel ist der von Beispiel 1 sehr ähnlich.

### Beispiel 5 (Wirkung einer Änderung des Vernetzungsmittels)

Es wurde die gleiche Herstellungsweise wie im Beispiel 1 gewählt, jedoch unter Verwendung von Diethanolamin anstatt Triethanolamin als Vernetzungsmittel. Der daraus resultierende hohe Anstieg der Viskosität der Lösung während des Umhüllungsvorganges führt zu einer eher teigigen Masse. Kristalle werden nur unzureichend mit dem Polymer umhüllt.

Qualitätstests wie für Beispiel 1 und 2 beschrieben ergaben, dass sich bereits nach 30 Minuten 80% des Acetamiprids in Lösung befinden.

### Beispiel 6 (Wirkung einer Änderung des Lösungsmittels)

In diesem Versuch wurde versucht, die Beschichtung der Acetamiprid-Kristalle unter Verwendung eines anderen Pflanzenölesters, Ethylhexyloleat (Radia^{®} 7331 der Fa. Oleon) als Lösungsmittel durchzuführen. Aufgrund unzureichender Mischbarkeit und wahrscheinlicher Reaktion des Polymerbildners mit diesem Lösungsmittel kam es zu keiner ausreichenden Beschichtung der Kristalle und der Versuch musste abgebrochen werden.

### Beispiel 7 (Beschichtung von Acetamiprid mit 18.89 g Polymer/100 g Acetamiprid)

Dieser Versuch basiert auf der Grundlage von Beispiel 1, wobei die Arbeitsschritte wie dort ausgeführt werden. Die Temperatur wird mit 60°C etwas höher gewählt.

| Komponente | Menge |
|---|---|
| Radia^{®} 7118 | 509,0 g |
| Agrimer^{®} AL 22 | 10,3 g |
| -- auf 60°C erhitzen -- | |
| Acetamiprid gemahlen @ 98,0% | 180,0 g |
| --- mit Ultra Turrax^{®} eindispergieren -- | |
| Polymerbildner Premix (siehe unten) | 47,5 g |
| -- langsam zugeben, 10 min rühren -- | |
| Vernetzer Premix (siehe unten) | 44,3 g |
| -- langsam innerhalb 30 min zugeben, intensiv rühren -- | |
| -- Temperatur 1 h auf 60°C halten -- | |
| Radia^{®} 7118 | 80,0 g |
| -- auf RT abkühlen lassen und über Nacht rühren -- | |
| Summe | 871.0 g |

| Polymerbildner Premix | Menge |
|---|---|
| Surfadonee^{®} LP 300 | 10,3 g |
| Voranate^{®} M220 | 34,0 g |
| Summe | 44,3 g |

| Vernetzer Premix | Menge |
|---|---|
| Surfadone^{®} LP 300 | 34,0 g |
| Triethanolamin (TEA) @ 100,0% | 13,4 g |
| Summe | 47,5 g |

### Beispiel 8 (Beschichtung von Acetamiprid mit 9,44g Polymer/100 g Acetamiprid)

Die Mengen an Polymerbildner Premix und Vernetzer Premix wurden gegenüber Beispiel 7 halbiert, sodass sich die rechnerische Beschichtungsstärke ebenfalls halbierte.

| Komponente | Menge |
|---|---|
| Radia^{®} 7118 | 554,9 g |
| Agrimer^{®} AL 22 | 10,3 g |
| -- auf 60°C erhitzen - | |
| Acetamiprid gemahlen @ 98,0% | 180,0 g |
| -- am Turrax^{®} eindispergieren -- | |
| Polymerbildner Premix (siehe unten) | 23,73 g |
| -- langsam zugeben, ca.10 min rühren | |
| Vernetzer Premix (siehe unten) | 22,1 g |
| --- langsam innerhalb von 15 min zugeben -- | |
| -- Temperatur 1 h auf 60-65°C halten -- | |
| Radia^{®} 7118 | 80,0 g |
| -- auf Raumtemperatur abkühlen lassen und über Nacht rühren -- | |

| | |
|---|---|
| Summe | 871.0 g |

| Polymerbildner Premix | Menge |
|---|---|
| Surfadonee^{®} LP 300 | 5,1 g |
| Voranate^{®} M220 | 17,0 g |
| Summe | 22,1 g |

| Vernetzer Premix | Menge |
|---|---|
| Surfadonee^{®} LP 300 | 17,0 g |
| Triethanolamin (TEA) @ 100,0% | 6,7 g |
| Summe | 23,7 g |

Das Beispiel der US 5 911 923 B1 wurde zu Vergleichszwecken nachgearbeitet, die erhaltende Dispersion von Mikrokapseln im gemäß US 5 911 923 B1 verwendeten Lösungsmittel (eine Mischung aus Toluol, Ethylacetat und Sojabohnenöl) erwies sich jedoch als nicht stabil, auch wiesen die erhaltenen Mikrokapseln nur eine geringe Wanddicke auf, so dass der Wirkstoff schnell hindurchdiffundieren konnte. Schon bei Verwendung des erfindungsgemäß bevorzugt verwendeten Lösungsmittels ergab sich hier eine wesentliche Verbesserung, Beispiel 9 zeigt das Verfahren gemäß der US 5 911 923 B1 unter Verwendung des erfindungsgemäß bevorzugten Lösungsmittels (jedoch ohne Verwendung von Diaminen als Vernetzungsmittel) mit einer bereits wesentlich verbesserten Verkapselung des verwendeten Wirkstoffs.

### Beispiel 9 (Beschichtung von Acetamiprid mit 18,89 g Polymer/100 g Acetamiprid)

Bei diesem Versuch wurde anstatt des erfindungsgemäß bevorzugten Aminoalkohols ein Diol (in diesem Fall Propylenglykol) verwendet, wobei die Polymerisationsreaktion in Anwesenheit eines Polymerisationskatalysators durchgeführt wird. Als Katalysator dient hier 1,4-Diazabicylo[2.2.2]octan, das üblicherweise bei der Herstellung von Polyurethanen verwendet wird. Die rechnerische Beschichtungsstärke ist gleich wie bei Beispiel 7.

| Komponente | Menge |
|---|---|
| Radia^{®} 7118 | 512,2 g |
| Agrimer^{®} AL 22 | 10,3 g |
| -- auf 60-65°C erhitzen -- | |
| Acetamiprid gemahlen @ 98,0% | 180,0 g |
| -- am Turrax^{®} gut eindispergieren -- | |
| Polymerbildner Premix (siehe unten) | 44,30 g |
| -- langsam zugeben und ca. 10 min rühren -- | |
| Vernetzer- Katalysator Premix (siehe unten) | 44,3 g |
| -- langsam bei 60-65°C zutropfen -- | |

| | |
|---|---|
| Summe | 791,0 g |
| Ausbeute | 791,0 ml |

| Polymerbildner Premix | Menge |
|---|---|
| Surfadone^{®} LP 300 | 10,3 g |
| Voranate^{®} M220 | 34,0 g |
| Summe | 44,3 g |
| Ausbeute | 44,3 ml |

| Vernetzer-Katalysator Premix | Menge |
|---|---|
| Surfadone^{®} LP 300 | 34,0 g |
| Propylenglykol | 9,5 g |
| DABCO | 0,8 g |
| Summe | 44,3 g |
| Ausbeute | 44,3 ml |

### Beispiel 10 (Vergleichsbeispiel, nicht erfindungsgemäße, klassische Mikroverkapselung nach dem o/w Emulsionsverfahren zum Erhalt von 16,00 g Polymer/100 g Acetamiprid)

Hierbei handelt es sich um eine nicht erfindungsgemäße, klassische Form der Wirkstoffverkapselung, wie sie ein Fachmann aufgrund der publizierten Literatur durchführen würde. Dabei wird der Aktivstoff Acetamiprid in einem mit Wasser nicht mischbaren Lösungsmittel gelöst (organische Phase) und in einem wässrigen Medium (Wasserphase) emulgiert, wobei der Nachteil darin besteht, dass das Wasser bereits während der Emulgierung einen Teil des Acetamiprides löst. Als Verkapselungspolymer wurde ein Polyisocyanat verwendet, welches mit einem Diamin als Vernetzer eine Grenzflächenpolymerisation eingeht und dabei eine Hülle aus Polyharnstoff bildet.

| Komponente | Menge |
|---|---|
| Wasserphase@50°C | 939 g |
| Ölphase@60°C | 610 g |
| Ethylendiamin Lösung 20% ig | 17 g |
| -- verkapseln mit Turrax^{®}-- | |
| -- 2h bei 50°C rühren -- | |
| Silfoam^{®} SRE, Antischaum von | |
| Wacker Chemie AG, DE | |
| ein Schaumstopp | 15,1 g |
| Glucopon^{®} 215 UP von BASF (DE), | |
| ein nicht-ionisches Surfactant | 56 g |
| ----Rest wurde weggelassen---- | |
| Proxel^{®} GXL von Lonza (IT), ein Biozid | 4,0 g |
| Citronensäure | 7,9 g |
| Wasser vollentsalzt | 126,6 g |
| Rhodopol^{®} von Brenntag (AT), | |
| Xanthangummi 23, 2%ig | 183 g |

| | |
|---|---|
| Summe | 1958 g |
| Ausbeute | 1958 ml |
| Ölphase | Menge |
| Purasolv^{®} EHL, 2-Ethylhexyl-L-lactat | 395 g |
| Voranate^{®} M 220 | 16 g |
| Acetamiprid | 100 g |
| Purasolv^{®} EHL | 99 g |
| Summe | 610 g |
| Ausbeute | 610 ml |

| Wasserphase | Menge |
|---|---|
| Wasser vollentsalzt | 713 g |
| Silfoam (TM) SRE | 0,5 g |
| Agrimer^{®} AL-10 LC | 11,9 g |
| Glucopon^{®} 215 UP | 3,7 g |
| PVP K-30^{®}, wasserlösliches Polyvinylpyrrolidon | 32 g |
| Silfoam^{®} SRE, Antischaum | 0,3 g |
| Wasser vollentsalzt | 178 g |
| Summe | 939 g |
| Ausbeute | 939 ml |

Die letzten 4 Beispiele 7 bis 10 sollen nun hinsichtlich ihrer Verkapselungsqualität verglichen werden. Hierbei wird die bereits im Zusammenhang mit der Qualitätskontrolle der Beispiel 1 und 2 erwähnte flüssige Bautenschutzfolie mit einem Gehalt von 2 g/kg Acetamiprid hergestellt und getrocknet. Die getrocknete Folie wird anschließend mit 100 ml Wasser extrahiert und der im Wasser gelöste Anteil Acetamiprid analytisch im Zeitverlauf bestimmt.

Qualitätskontrolle zu Beispiel 7 (erfindungsgemäße Ausführung mit TEA und 18,89 g Polymer/100g Acetamiprid):

| Extraktionszeit | Einwaage Probe [g] | Gehalt in der Folie [g/kg] | Acetamiprid in Lösung [mg/100ml] | Acetamiprid gelöst |
|---|---|---|---|---|
| 30 Minuten | 6,4 | 2,2 | 1,085 | 8% |
| 24 Stunden | 6,4 | 2,2 | 1,8 | 13% |
| 48 Stunden | 6,4 | 2,2 | 2,2 | 16% |

Qualitätskontrolle zu Beispiel 8 (erfindungsgemäße Ausführung mit TEA; 9,44 g Polymer/100g Acetamiprid)

| Extraktionszeit | Einwaage Probe [g] | Gehalt in der Folie [g/kg] | Acetamiprid in Lösung [mg/100ml] | Acetamiprid gelöst |
|---|---|---|---|---|
| 30 Minuten | 5,7 | 2 | 10,5 | 92% |
| 24 Stunden | 5,7 | 2 | 10,5 | 92% |
| 48 Stunden | 5,7 | 2 | - | - |

Qualitätskontrolle zu Beispiel 9 (Ausführung mit Diol und DABCO; 18,89 g Polymer/100g Acetamiprid)

| Extraktionszeit | Einwaage Probe [g] | Gehalt in der Folie [g/kg] | Acetamiprid in Lösung [mg/100ml] | Acetamiprid gelöst |
|---|---|---|---|---|
| 30 Minuten | 5,4 | 2,1 | 5,3 | 47% |
| 24 Stunden | 5,4 | 2,1 | 5,7 | 50% |
| 48 Stunden | 5,4 | 2,1 | 6,2 | 55% |

Qualitätskontrolle zu Beispiel 10 (Vergleichsbeispiel) (nicht erfindungsgemäße Ausführung mit Emulsionsverfahren, 16,00 g Polymer/ 100 g Acetamiprid)

| Extraktionszeit | Einwaage Probe [g] | Gehalt in der Folie [g/kg] | Acetamiprid in Lösung [mg/100ml] | Acetamiprid gelöst |
|---|---|---|---|---|
| 30 Minuten | 5,3 | 2,1 | 10 | 90% |
| 24 Stunden | 5,4 | 2,1 | - | - |
| 48 Stunden | 5,4 | 2,1 | - | - |

Es zeigt sich dabei deutlich, dass bei der erfindungsgemäßen Ausführung der Beschichtung der Wirkstoff deutlich langsamer freigesetzt wird als bei einer klassischen, nicht erfindungsgemäßen Ausführung. Dabei tritt im Falle der Verwendung von Acetamiprid als Wirkstoff eine für den Zweck der Bautenschutzfolie brauchbare Lösungsverzögerung ab einer Menge von etwa 19 g Polymerbildner pro 100 g Acetamiprid ein. Eine Halbierung der Polymerbildner Menge führt wie in Beispiel 3 und 8 gezeigt zu keiner brauchbaren Lösungsverzögerung mehr.

Die für den jeweiligen Einsatzzweck optimale Menge an Polymerbildner richtet sich unter anderem nach der Form und mittleren Teilchengröße des Beschichtungssubstrates und es obliegt dem Fachmann, diese durch Berechnungen und Versuche für den jeweiligen Zweck festzulegen.

## Patentansprüche

1. Verfahren zur Herstellung von Konzentraten von vorzugsweise wasserlöslichen Wirkstoffen, **dadurch gekennzeichnet, dass** in einem wasserfreien Verfahren unter Verwendung fester Wirkstoffe als Ausgangsmaterial die Kristalle eines Wirkstoffs in einem ersten organischen Lösungsmittel unter Zugabe eines Dispergierhilfsmittels gleichmäßig verteilt werden, gegebenenfalls die Viskosität der so erhaltenen Lösung mit einem geeigneten Hilfsmittel eingestellt wird, zu der derart erhaltenen Lösung ein Polymerbildner, gegebenenfalls in einem zweiten organischen Lösungsmittel, zugegeben wird, wobei die Viskosität entweder der zuzugebenden oder der erhaltenen Lösung gegebenenfalls durch Zugabe eines geeigneten Hilfsmittels eingestellt wird, und der erhaltenen Lösung ein Vernetzungsmittel mit mindestens zwei funktionellen Gruppen in einem dritten organischen Lösungsmittel zurückgegeben wird, wobei wiederum die Viskosität entweder der zuzugeben oder der erhaltenen Lösung gegebenenfalls durch Zugabe eines geeigneten Hilfsmittels eingestellt wird.

2. Verfahren nach Anspruch 1, **dadurch gekennzeichnet, dass** der Wirkstoff ausgewählt ist aus der Gruppe umfassend biologisch wirksame, insektizide, herbizide, fungizide, akarizide, algizide, mikrobizide, mikrobistatische, rodentizide und antibiotisch wirksame Wirkstoffe, Repellentien, Attraktoren, Pheromone und allgemein attraktiv oder abwehrend wirkende Duft- oder Geschmacksstoffe sowie Mischungen derartiger Wirkstoffe.

3. Verfahren nach Anspruch 1 oder 2, **dadurch gekennzeichnet, dass** das Dispergierhilfsmittel ein Polyvinylpyrrolidonderivat ist.

4. Verfahren nach einem der Ansprüche 1 bis 3, **dadurch gekennzeichnet, dass** das bzw. die Lösungsmittel ausgewählt ist/sind aus der Gruppe umfassend Ester von Pflanzenölen, insbesondere Methylester und Ethylhexylester der mittelkettigen gesättigten, allenfalls einfach oder mehrfach ungesättigten Fettsäuren mit 8 bis 16 C-Atomen ohne reaktive Hydroxylgruppen, Terpenkohlenwasserstoffe, insbesondere Orangenterpen, aliphatische und aromatische Kohlenwasserstoffe, Ether und Ester natürlicher und technischer aliphatischer und aromatischer Alkohole in verzweigter oder unverzweigter Form und Mischungen hievon.

5. Verfahren nach einem der Ansprüche 1 bis 4, **dadurch gekennzeichnet, dass** der Polymerbildner ausgewählt ist aus der Gruppe umfassend niedrigviskose Polymethylen-Polyphenylisocyanate, vorzugsweise mit einem mittlerem NCO Gehalt von 25-35, besonders bevorzugt 30 - 32%, vorzugsweise niedrigviskose Toluoldiisocyanate mit geringer Acidität, sowie Mischungen hievon.

6. Verfahren nach einem der Ansprüche 1 bis 5, **dadurch gekennzeichnet, dass** die Umsetzung bei zwischen 10 und 80, vorzugsweise zwischen 40 und 60°C stattfindet, bzw. bei einer Temperatur von mindestens 1 °C, besser mindestens 5 °C, besonders bevorzugt mehr als 10°C unterhalb der Flammtemperatur des jeweils verwendeten organischen Lösungsmittels oder/und einer Temperatur, bei welcher das bzw. die verwendete(n) Lösungsmittel einen derart geringen Dampfdruck aufweisen, dass bei Reaktionsbedingungen keine explosionsgefährliche Atmosphäre entsteht, stattfindet.

7. Verfahren nach einem der Ansprüche 1 bis 6, **dadurch gekennzeichnet, dass** das Vernetzungsmittel ein zumindest bifunktioneller Quervernetzer für NCO Gruppen ist und in zumindest stöchiometrischer Menge zugegeben wird, wobei das Vernetzungsmittel vorzugsweise ein Ethanolamin, insbesondere Triethanolamin (TEA) ist oder umfasst.

8. Pulver oder Granulat, umfassend beschichtete Kristalle eines Wirkstoffs erhältlich nach einem Verfahren nach einem der Ansprüche 1 bis 7.

## Geänderte Patentansprüche

### Geänderte Patentansprüche gemäss Regel 137(2) EPÜ.

1. Verfahren zur Herstellung von Konzentraten von wasserlöslichen Wirkstoffen, **dadurch gekennzeichnet, dass** in einem wasserfreien Verfahren unter Verwendung fester Wirkstoffe als Ausgangsmaterial die Kristalle eines Wirkstoffs in einem ersten organischen Lösungsmittel unter Zugabe eines Dispergierhilfsmittels gleichmäßig verteilt werden, gegebenenfalls die Viskosität der so erhaltenen Lösung mit einem geeigneten Hilfsmittel eingestellt wird, zu der derart erhaltenen Lösung ein Polymerbildner, gegebenenfalls in einem zweiten organischen Lösungsmittel, zugegeben wird, wobei die Viskosität entweder der zuzugebenden oder der erhaltenen Lösung gegebenenfalls durch Zugabe eines geeigneten Hilfsmittels eingestellt wird, und der erhaltenen Lösung ein Vernetzungsmittel mit mindestens zwei funktionellen Gruppen in einem dritten organischen Lösungsmittel zurückgegeben wird, wobei wiederum die Viskosität entweder der zuzugeben oder der erhaltenen Lösung gegebenenfalls durch Zugabe eines geeigneten Hilfsmittels eingestellt wird und der Polymerbildner ausgewählt ist aus der Gruppe umfassend niedrigviskose Polymethylen-Polyphenylisocyanate, vorzugsweise mit einem mittlerem NCO Gehalt von 25-35, besonders bevorzugt 30 - 32%, sowie Mischungen hievon.

2. Verfahren nach Anspruch 1, **dadurch gekennzeichnet, dass** der Wirkstoff ausgewählt ist aus der Gruppe umfassend biologisch wirksame, insektizide, herbizide, fungizide, akarizide, algizide, mikrobizide, mikrobistatische, rodentizide und antibiotisch wirksame Wirkstoffe, Repellentien, Attraktoren, Pheromone und allgemein attraktiv oder abwehrend wirkende Duft- oder Geschmacksstoffe sowie Mischungen derartiger Wirkstoffe.

3. Verfahren nach Anspruch 1 oder 2, **dadurch gekennzeichnet, dass** das Dispergierhilfsmittel ein Polyvinylpyrrolidonderivat ist.

4. Verfahren nach einem der Ansprüche 1 bis 3, **dadurch gekennzeichnet, dass** das bzw. die Lösungsmittel ausgewählt ist/sind aus der Gruppe umfassend Ester von Pflanzenölen, insbesondere Methylester und Ethylhexylester der mittelkettigen gesättigten, allenfalls einfach oder mehrfach ungesättigten Fettsäuren mit 8 bis 16 C-Atomen ohne reaktive Hydroxylgruppen, Terpenkohlenwasserstoffe, insbesondere Orangenterpen, aliphatische und aromatische Kohlenwasserstoffe, Ether und Ester natürlicher und technischer aliphatischer und aromatischer Alkohole in verzweigter oder unverzweigter Form und Mischungen hievon.

5. Verfahren nach einem der Ansprüche 1 bis 4, **dadurch gekennzeichnet, dass** die Umsetzung bei zwischen 10 und 80, vorzugsweise zwischen 40 und 60°C stattfindet, bzw. bei einer Temperatur von mindestens 1 °C, besser mindestens 5 °C, besonders bevorzugt mehr als 10°C unterhalb der Flammtemperatur des jeweils verwendeten organischen Lösungsmittels oder/und einer Temperatur, bei welcher das bzw. die verwendete(n) Lösungsmittel einen derart geringen Dampfdruck aufweisen, dass bei Reaktionsbedingungen keine explosionsgefährliche Atmosphäre entsteht, stattfindet.

6. Verfahren nach einem der Ansprüche 1 bis 5, **dadurch gekennzeichnet, dass** das Vernetzungsmittel ein zumindest bifunktioneller Quervernetzer für NCO Gruppen ist und in zumindest stöchiometrischer Menge zugegeben wird, wobei das Vernetzungsmittel vorzugsweise ein Ethanolamin, insbesondere Triethanolamin (TEA) ist oder umfasst.

7. Pulver oder Granulat, umfassend beschichtete Kristalle eines Wirkstoffs erhältlich nach einem Verfahren nach einem der Ansprüche 1 bis 6.
